# EUROPEAN PATENT APPLICATION

(11) **EP 2 347 764 A2**
(43) Date of publication of application: **27.07.2011**
(21) Application number: 10185705.0
(22) Date of filing: 27.11.2003
(51) Int. Cl.: A61K 39/095, A61K 39/008, A61K 39/39, A61K 39/00, A61K 39/02

(54) **Method of obtaining cochlear structures, vaccine compositions, adjuvants and intermediates thereof**

(30) Priority: 27.11.2002 CU 20020292
(62) Divisional of application: 03773445.6
(71) Applicant: Instituto Finlay, Centro de Investigacion-Produccion de vacunas y sueros, La Coronela, La Lisa, Ciudad Habana 13500 (CU)
(72) Inventor: Perez Martin, Oliver Germán, 11600 Ciudad de la Habana (CU); Bracho Grando, Gustavo Rafael, 11500 Ciudad de la Habana (CU); Lastre Gonzalez, Miriam de San Juan Bosco, 11600 Ciudad de la Habana (CU); Sierra Gonzalez, Victoriano Gustavo, 16017 Ciudad de la Habana (CU); Campa Huergo, Concepcion, 12100 Ciudad de la Habana (CU); Mora Gonzalez, Nestor, 222096 C. de Avila, Ciudad de Habana (CU); Barbera Morales, Ramon Faustino, 12100 Ciudad de la Habana (CU); Del Campo Alonso, Judith Monica, 10400 Ciudad de la Habana (CU); Rodriguez Ramirez, Tamara, 11200 Ciudad de la Habana (CU); Zayas Vignier, Caridad, 11600 Ciudad de la Habana (CU); Gil Martinez, Danay, 11600 Ciudad de la Habana (CU); Taboada Suarez, Carlos, Ciudad de la Habana (CU)
(74) Representative: Jansen, Cornelis Marinus

(57) **Abstract**

The present invention has applications in the field of immunology, specifically in the sphere of adjuvants and vaccines. The invention aims at obtaining cochlear structures from vesicles found in the outer membranes of microorganisms, in order to employ them in the preparation of adjuvants and vaccines. The invention also discloses a method for obtaining cochlear structures.

## Description

### DESCRIPTIVE MEMORY

The present invention has applications in the field of immunology, specifically in the sphere of adjuvants and vaccines.

In the search for efficient vaccines, finding adequate antigens has proved a challenge in various spheres of knowledge beyond vaccinology. Once the appropriate antigen has been isolated, the latter often yields an immunological effect that is insufficient for the purposes at hand, or does not induce the desired effects, demanding the application of adequate adjuvants. Elaborating vaccines for diseases for which there is currently no protection, as well as improving existing vaccines and developing strong adjuvants for use in multiple and new generation vaccines, constitutes, to this day, an urgent necessity. So does developing vaccines which include different antigens and prove effective both in the treatment of adults and children, and, more importantly, in that of newborns, as well as finding adjuvants that function at the level of the mucosa and are capable of resisting the acidic contents of the stomach.

Mucosal immunization is an increasingly widespread vaccinological practice, as many microorganisms penetrate the body through mucous membranes. The mucosa present a number of particularities, including: the existence of a common mucous system (allowing us to induce local as well as distant responses), and the fact that Ig (immunoglobulin) A is the chief antibody employed in its immunological defense mechanisms.

This type of immunization also offers a number of advantages, including an uncomplicated administration that does not require the use of syringes, a lower cost of production and a lower level of reactogenesis, which renders it all the much safer with respect to parenteral vaccines and the induction of both mucosal and systemic reactions.

Mucosal immunization, however, meets with a number of obstacles: the stomach's acidic contents, which reach extreme acidic pH levels; the duodenum's basicity and the peristalsis of the digestive tract, that, in combination with the action of M cells from inductor organs working at the level of the mucosa, specialized in the sampling of antigens, reduces the efficacy of the antigens in the vaccine. The cilia and mucous formations in the respiratory mucosal organs also interfere with the sampling of the antigens by the M cells.

The present strategy utilized to avoid the contact of the antigen with an acidic pH medium consists in the administration of vaccines in bicarbonated solutions at times far removed from mealtimes, so as to reduce the acidity of the stomach and ensure a quick passage through it (Benitez JA et al., Infect and Immun 1999,67(2):539-545), or the coating of the antigens with agents resistant to acidic substances, such as liposomes.
Currently, the methods used in the preparation of liposomes, and in the coating of solid liposoluble materials, are well known (Schneider U.S. Pat. No. 4, 089, 801, Ash et al., U.S. Pat No. 4, 448, 765 and Miller et al., U.S. Patent No. 4, 133, 874). The main problems posed by the encapsulation of pharmaceutical materials in liposomes include: the little stability it shows in laboratory trials; the spilling of the encapsulated material; the reduction of the drug's efficacy; the susceptibility shown to adverse environmental conditions, digestion in the gastro-intestinal tract and indirect fusion with cellular membranes (httg://www.BDSiAdvantages.html). On the other hand, liposomes are unstable structures that, for the most part, cannot be freeze dried, a problem which has been overcome by the development of cochlear structures.

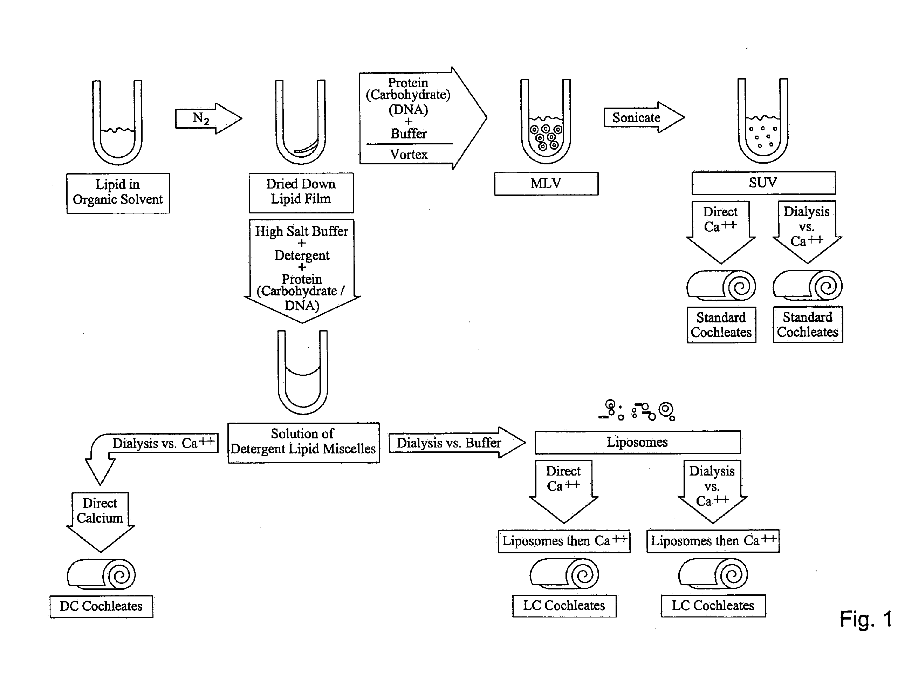

Cochleates are multi-laminary lipidic structures rolled up in the forms of sea-shells. The production of cochleates through the fusion of uni-laminary liposomes and the employment of divalent cations, is a well known practice (D. Papahadjopolous et al., Biochem. Biophys. Acta, 1975; 394:483). This procedure has been modified to produce a suspension of lipidic multi-laminary vesicles containing and surrounded by the antigen. The latter is converted into small, uni-laminary lipidic proteic vesicles through sonication under nitrogen, which is done in order to form the cochleates in the presence of divalent ions (Gould-Fogerite et al. U. S. Pat. No 5, 643, 574, July 1, 1997). These techniques are summarized in Fig. 1.

Cochleates, as well as other auto-assembled micro-structures, have been employed in the administration of therapeutic agents (Yager, et al. U.S. Pat. No. 5, 851, 536, December 22, 1998, Gould-Forgerite, et al. U.S. Pat. No. 5, 994, 318, November 30, 1999 and Yager, et al. U.S. Pat. No. 6, 180, 114, January 30, 2001). These include preparations of cochleates containing adjuvants (Gould-Fogerite et al. U. S. Pat. No 5, 994, 318, November 30, 1999). Nevertheless, both liposomes and cochleates must be derived from negative lipids in the presence of cholesterol, both of which are generally extracted from animals in costly procedures (Mannino et al. U.S. Pat. No. 4, 663, 161. May 5, 1987) that are increasingly less acceptable in light of new pharmaceutical regulations and that preferably include a purified protein obtained from a microorganism, or a peptide, in the case of vaccine preparations. Furthermore, we should point out that the use of cochleates as adjuvants in their own right, or their preparation with other activators of important signals in the inducement of immunological responses, such as molecular structures associated with pathogens (phylogenetically preserved structures for which there are receptors in the host, and which are recognized as a sign of danger by that host), has not been previously considered.

The aim of the present invention is to obtain a new cochlear structure from vesicles found in the outer membranes of live organisms, which present adjuvant and vaccinal properties due to their particular protein and lipidic make-up, as well as to the molecular structures associated to pathogens found in the organism. Once formed, the cochlear structures are homogenized at their own size to make them more immunologically effective.

The cochlear structures obtained through the present invention are characterized by the fact that, having a proteolipidic composition as yet untried by other authors, they are capable of auto-assembling themselves and of producing rolled, sea-shell shaped structures. The protein and lipidic compositions of the cochlear structures will depend on the microorganism that has supplied the vesicles of its outer membrane, that is, it will depend on the characteristics of the proteins found in its membrane. In the same fashion, the mentioned structures contain concentrations of molecular structures associated to pathogens, between 1 and 7 % in relation to the concentration of proteins, supplied by the membrane of the microorganism in question, which are inserted and are not found within it in a free state. Furthermore, these structures can be purified from other microorganisms and can be added to the preparation. The added and existing structures must be at a concentration between 1 and 30 % in relation to the concentration of proteins. One of the molecular structures associated to pathogens utilized during the production of cochlear structures was the lipopolysaccharide of *Vibrio cholerae* or *N. meningitidis* (example 20).

The cochlear structures obtained through the invention induced a cellular response that proved effective in breastfed infants, showed properties of thermal as well as acidic and basic resistance, and - overall - proved well-suited for mucosal administration (examples 2, 4, 6, 8 and 10). These properties were useful in the design of heterologous adjuvants (adjuvants employed to strengthen vaccines different from those that produced the outer membrane vesicles) and homologous vaccines (vaccines used against the microorganism that has supplied the outer membrane vesicles), employing the mentioned structures.

With respect to the vaccine composition containing said structure, it is important to point out that the response of the serums was superior, at various times of the experiment, to that obtained while employing a vaccine based on vesicles of outer membrane with an aluminum oxide adjuvant, known in the market as VAMENGOC-BC^{®}, and that it also induced immunoglobulin A specific on being administered through the mucosa. Furthermore, the cochlear structures stimulated CD8 lymphocytes, an important part of all immunological responses to intra-cellular organisms.

The adjuvant action of the cochlear structures was evaluated through various tests, including: the production of IL12 in the human histiocytary cell line U937 (example 11) and the production of nitric oxide in the murine macrophagyc cell line J774, in the absence of all stimuli (example 13); the stimulation of human dendrites (example 15) and the reduction in the induration of lesions in the challenge experiment, "challenging" mice immunized with cochlear structures containing antigens derived from this protozoan organism with *Leishmania major* (example 16).
The cochlear structures produced through the invention make the resulting adjuvant or vaccine induce earlier, stronger and more durable responses "in vivo", while an efficient induction of mediators involved in the induction of a cellular structure, and a good stimulation of cells presenting professional antigens (dendritic cells), was observed "in vitro" (examples 11, 13, and 15).

Another aim of the present invention is the use of vesicles found in the outer membrane of microorganisms, which constitute the starting point for the formation of cochlear structures, as vaccines or as heterologous adjuvants, such that eliminating the adsorption of aluminum hydroxide does not limit the immunogenic capabilities of the former. It is worth mentioning that the capacity of these vesicles to induce parenteral responses all by themselves has not been sufficiently investigated.

Said vesicles are known as auto-assembled nanospheres, and are constituted by a lipidic bi-layer with proteins and polysaccharides inserted in it. These can be extracted from any pathogen and may present different molecular structures (especially lipopolysaccharide, peptidoglycan, lipoprotein, teicoic acid, flagellin or lipophosphoglycan). Lipophosphoglycan and lipopolysaccharide were obtained from *Leishmantia major* and *N. meningitidis* or *Salmonella tiphy,* respectively, during the process of obtaining the vesicles from the outer membranes of the organisms, remaining inserted in the former and never in a free state, in proportions between 1 and 7 % of the protein weight.

Used in the vaccine preparations, the outer membrane vesicles extracted from *Salmonella tiphy* or from *N. Meningitidis* B induced a response from the IgA when inoculated nasally, and a good immunological response when parenterally inoculated (examples 3, 5, 7, 12 and 14).

The adjuvant effect of the outer membrane vesicles was evaluated through a number of tests, including: the production of IL12 in the human histiocytary line U937 (example 12) and the production of nitric oxide in the murine macrophagic line J774 in the absence of other stimuli (example 14); the strengthening of the cellular response (an increase of IgG2a) through the combination of polysaccharides with vesicles found in the outer membrane of *N. meningitides,* in comparison to its fusion with a tetanus toxoid (example 18) and the strengthening of the response of antibodies reactive against polysaccharides Vi from *S. Tiphy* through combination with vesicles of outer membrane found in the same bacteria (example 19).

The use of cochlear structures and outer membrane vesicles as adjuvants or vaccines resulted in an unexpected strengthening of the immune response induced through prior doses of vaccines or through contact with the germ. It is important to point out that the latter were administered differently than those derived from cochlear structures or vesicles (example 22).

The invention also discloses a method for obtaining cochlear structures from the vesicles found in the outer membranes of live organisms. The following steps are taken to achieve this: In the first place, the outer membrane, which is structured into vesicles by live microorganisms or cells, is purified using any of the methods widely employed by experts in the field. The preferred methods are those disclosed in EP 301992, US 5,597,572, EP 11243 or US 4,271,147, Zollinger et al. (J. Clin. Invest. 1979, 63:836-848), Frederikson et al. (NIPH Annals 1991, 14: 67-80), Sauders et al. (infect. Immun. 1999,67:113-119), Drabick et al. (Vaccine 2000, 18:160-172), WO 01/09350 or EP 885900077.8 and US 5,597,572. The membranes are so purified that they contain between 1 and 7 % of lipopolysaccharide, completely inserted into the vesicle. A solution of a total protein concentration between 3 and 6 mg/mL is prepared, increasing the concentration of non-ionic detergent to 8 to 12 times that of the protein concentration, in order to completely dissolve the vesicles. This solutions is subsequently sterilized by filtration through a membrane with a pore size of 0.2 µm, in which the vesicle aggregates which had not been dissolved are also eliminated. Following this, a rotational dialysis or tangential filtration is carried out. The dialysis is carried out for 24 hours against a solution containing adequate concentrations of a multivalent ion (particularly Ca²⁺, Zn²⁺ or Mg²⁺, at concentrations ranging from 2.5 to 6.5 mM) at a pH condition of pH 7.4 ± 0.2. Finally, the cochlear structures obtained are submitted to a mechanical treatment (sonication in a water bath between 15 °C and 25 °C in temperature for 45 minutes, in particular), in order to homogenize the particle sizes.

This constitutes a rapid and efficient method for obtaining cochlear structures which contain multiple proteins and lipids from the outer membrane of the microorganism employed, as well as molecular structures associated to pathogens naturally obtained. These structures demonstrate a high level of stability and immunogenicity.

On the other hand, the uncomplicated and efficient method for obtaining them permits us to introduce new antigens to the mentioned structure. The new antigens are added to the suspension of vesicles of outer membrane prepared for obtaining said structures, after increasing the concentration of detergents and prior to the addition of the multivalent ions during the process of dialysis. Among the antigens that may be added are the saccharides, lipoproteins, peptides, conjugates and nucleic acids. These must be at a concentration between 0.2 to 2.7 µg for every 3 to 9 µg of proteins. It is also possible to incorporate other molecular structures associated to pathogens to stimulate the innate and adquired response, something which renders it useful as heterologous adjuvants. The lipopolysaccharide of *Vibrio cholerae,* amastygotes or promastygotes of *Leishmania mayor*, were the structures especially employed, which allowed us to induce cellular responses as well as the activity of antibodies reactive against them. In addition to this, plasmidic DNA, containing fluorescent-green protein, was introduced and placed against macrophagyc lines; the molecule's fluorescence later allowed us to determine its intracellular presence. An alergenic derived from *Dermatophagoides siboney* was also introduced and the resulting cellular response induced was determined (examples 16 and 17).

The use of live organisms as a source of raw material for obtaining cochlear structures has not been described by any author. Neither has the process of incorporating one or more molecular structures associated to pathogens into the cochlear structures, as in the case of the present invention.

The invention shall be described through the following, specific examples.

### Example 1. Obtaining the cochlear strucutres

We began with the vesicles extracted from the outer membranes of microorganisms using the methods described in EP 885900077.8 or US 5,597,572. These were resuspended in a buffer solution of Tris-EDTA with 0.5 % of sodium deoxycholate. The protein concentration of the suspension was determined using Lowry's method, modified by Peterson (Analyt. Biochem. 83,346,1977). The phospholipid content of the vesicles was determined by determining the content of inorganic phosphorous (Bartlett, J Biol. Chem 234, 466, 1959). Both the protein and phospholipid concentrations were employed to determine the optimal conditions and the amount of detergent needed for the formation of the cochlear structures. A solution containing the vesicles was prepared, adjusted to a final protein concentration of 5-6 mg/mL in a Tris-EDTA buffer containing sodium deoxycholate at a concentration 6 to 15 times that of the total concentration of proteins. This solution was filtered in the dialysis apparatus using a filter with a pore size of 0.2 µm. The dialysis was carried out using the rotational agitation method for a period of 24 hours, with a continuous and slow change of the dialysis buffer. This last solution was made up of NaCl 50 - 150 mM, Imidazol 1-4 mM, HEPES 3-5 mM and CaCl 2-7 mM in H₂O prepared in sterile conditions that were preserved throughout every step of the procedure. The formation of cochlear structures was confirmed by the appearance of a white precipitate and by subsequent optical and electronic microscopic observations. The concentration of proteins and phospholipids was once again calculated and adjusted for subsequent trials. The physical and chemical properties of the proteins included in the cochlear structures were checked and compared with that of the vesicles through electrophoresis in polyacrylamide gels tinted with Coomassie Blue.

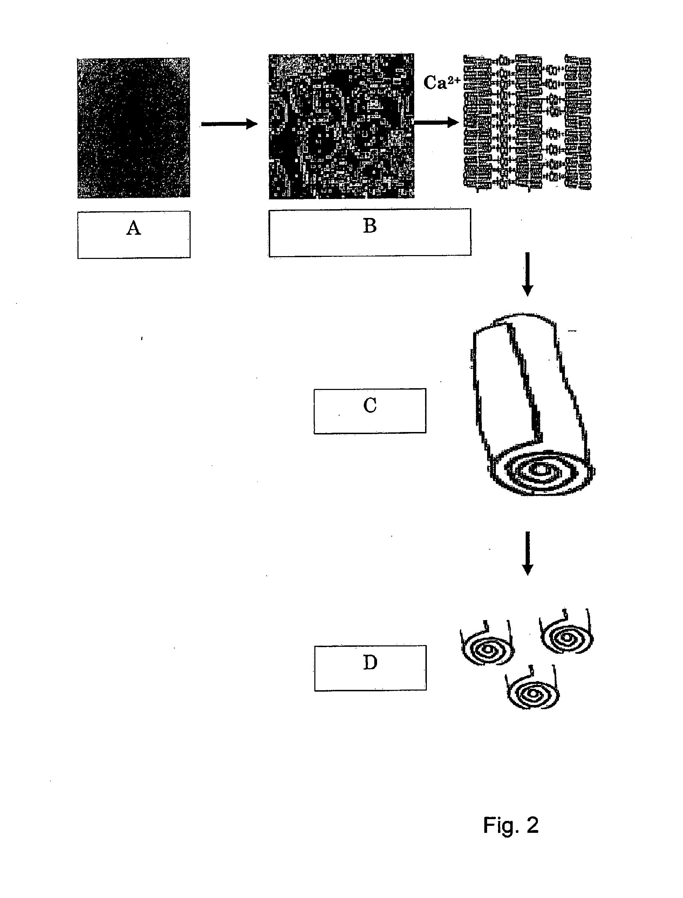

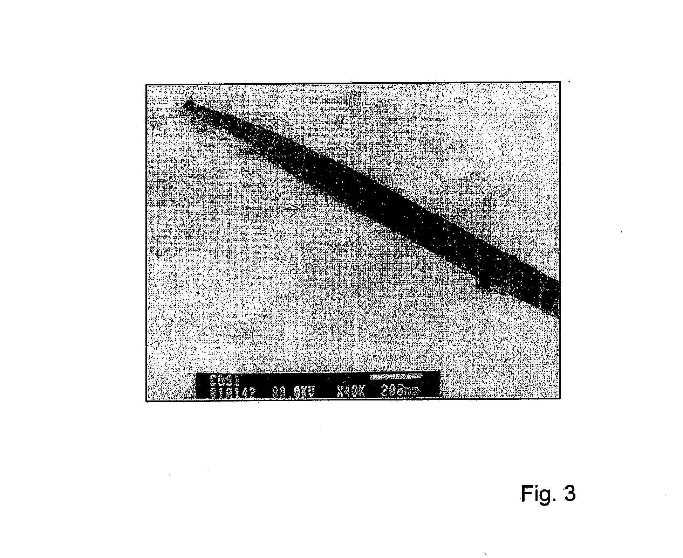

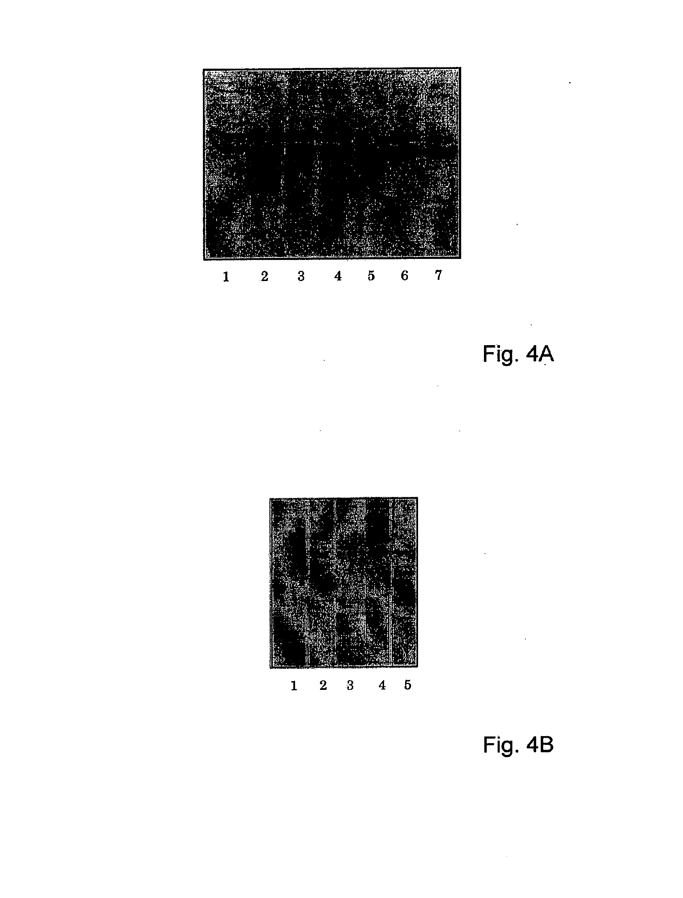

The structural integrity of the latter was determined and confirmed using the Western Blot method (Fig. 2 - 4).

### Example 2. Responses induced by the cochlear structures in mice, compared to those induced by the vaccine VA-MENGOC-BC®.

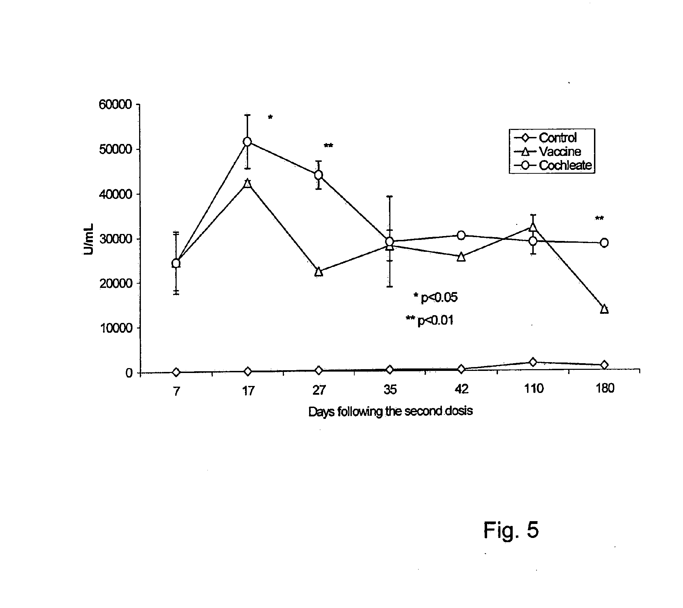

Balb/c mice were intra-muscularly immunized with 12 µg of proteins per mice, in 2 doses separated by 21 days, with VA-MENGOC-BC^{®} or cochlear structures. Blood samples were taken from the animals at the indicated times following the second dose and the seric responses of IgG against outer membrane vesicles were evaluated through an ELISA test. Significant differences (p<0.05) were observed between the responses induced by the cochlear strucutres and the vaccine, always in favor of the former, at 17, 27 and 180 days following the second dose (Fig. 5)

### Example 3. Responses induced in mice by parenterally administered vesicles of outer membrane, in comparison to those induced by the vaccine VA-MENGOC-BC®.

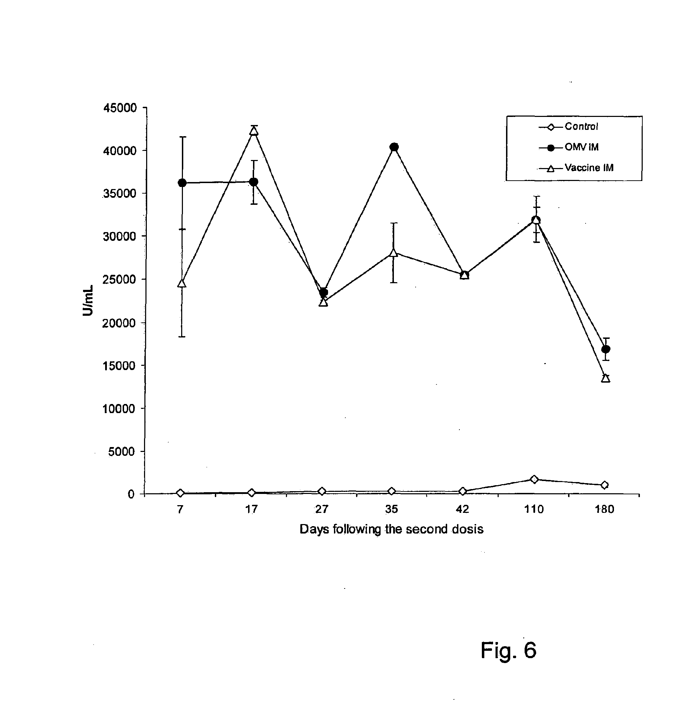

Balb/c mice were intra-muscularly immunized with 12 µg of proteins per mice in two doses separated by 21 days, with VA-MENGOC-BC^{®} or outer membrane vesicles. Blood samples were taken from the animals at the indicated times following the second dose and the seric responses of IgG against the vesicles were evaluated through an ELISA test. No significant differences (p<0.05) were observed between the responses induced by the vesicles and those induced by the vaccine. These results confirm the usefulness of the vesicles and vaccines in their own right (Fig. 6)

### Example 4. Effectiveness of nasal (IN) or gastric (IG) immunization with cochlear structures

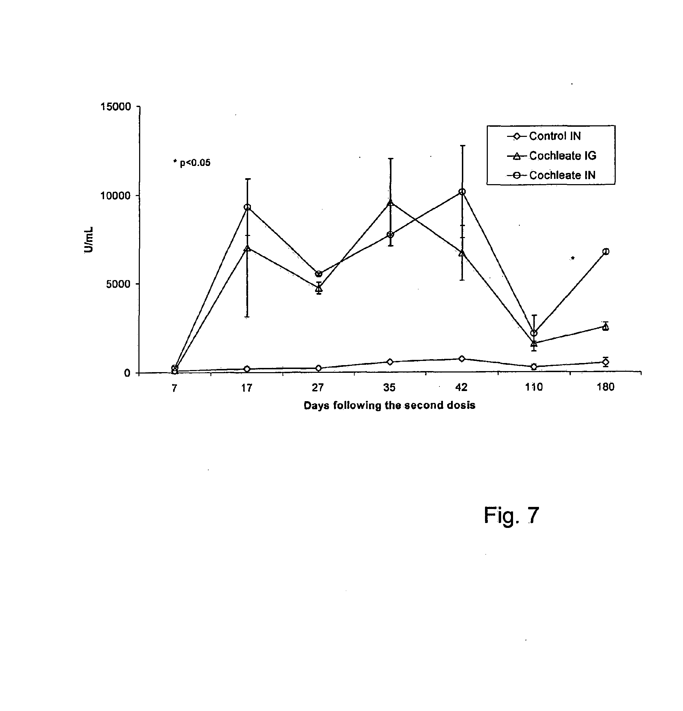

Balb/c mice were intra-nasally (IN) or intra-gastrically (IG) immunized with 100 or 12 µg of proteins per mice, in two doses separated by 21 days, respectively. Blood samples were taken from the animals at the indicated times after the second dose and the seric responses of IgG against the vesicles were evaluated through an ELISA test. Good responses from the IgG were induced against the outer membrane vesicles with both concentrations of cochlear structures intra-nasally and intra-gastrically inoculated. This suggests that good systemic responses are to be gotten through mucosal inoculation (Fig. 7).

### Example 5. Effectiveness of nasal immunization (IN) with outer membrane vesicles

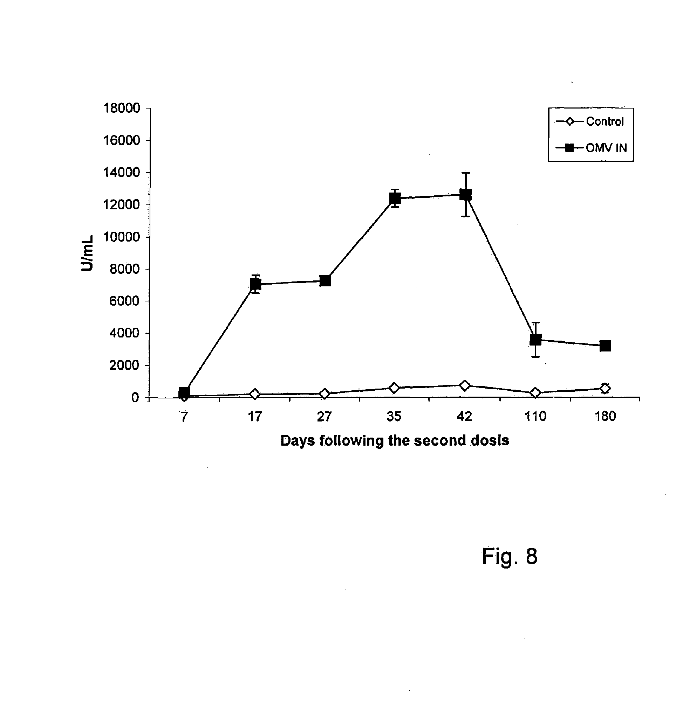

Balb/c mice were intra-nasally (IN) immunized with 12 µg of proteins per mice, in two doses separated by 21 days. Blood samples were taken from the animals at the indicated times after the second dose and the seric responses of IgG against the vesicles were evaluated through an ELISA test. Good IgG responses against the vesicles were obtained through this inoculation method, suggesting that valuable systemic responses can be obtained through intra-nasal inoculation (Fig. 8)

### Example 6. Effectiveness of intra-nasally or intra-gastrically administered cochlear structures in inducing IgA in saliva

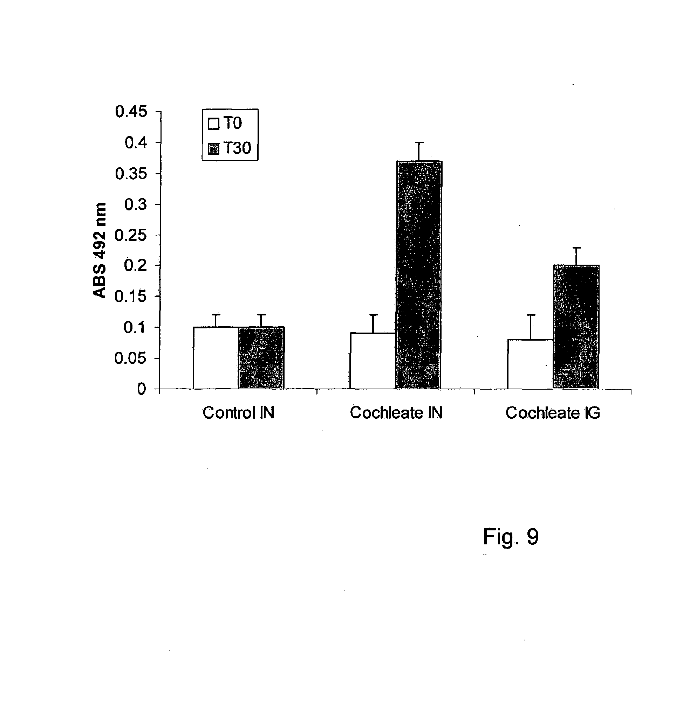

Balb/c mice were intra-nasally (IN) or intra-gastrically (IG) immunized with 100 or 12 µg of proteins per mice, in two doses separated by 21 days, respectively. Saliva samples were taken from the animals 9 days after the last dose was administered and the response of IgA against outer membrane vesicles was evaluated through an ELISA test. Significant responses of IgA against the vesicles were obtained using the IN method and a small but important increase in the IgA anti-vesicles was obtained using the IG method (Fig. 9).

### Example 7. Effectiveness of intra-nasally administered outer membrane vesicles in inducing IgA in saliva

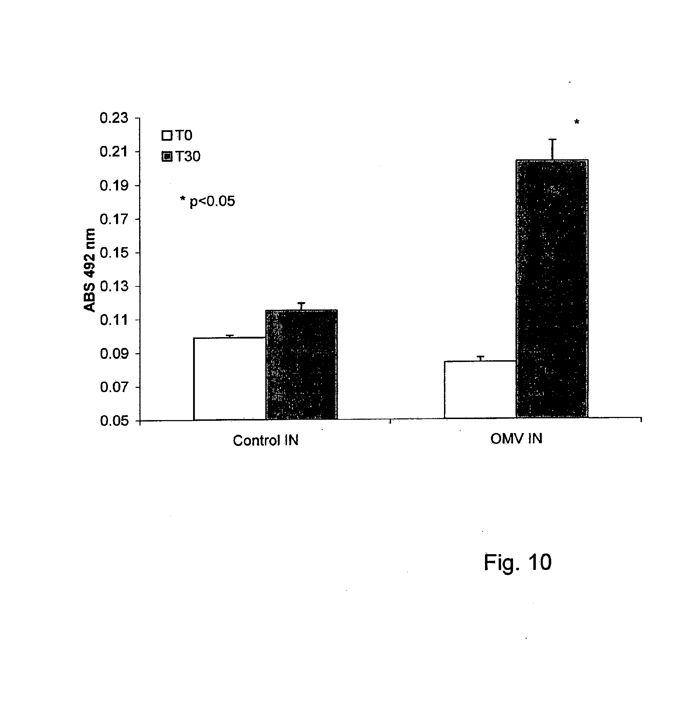

Balb/c mice were intra-nasally (IN) immunized with 12 µg of proteins per mice, in two doses separated by 21 days. Saliva samples were taken from the animals 9 days after the last dose was administered and the response of IgA against outer membrane vesicles was evaluated through an ELISA test. Significant responses of IgA against the vesicles were obtained using the IN method (Fig. 10).

### Example 8. Subclasses of IgG reactive against vesicles of outer membranes in serum induced through immunization with cochlear structures

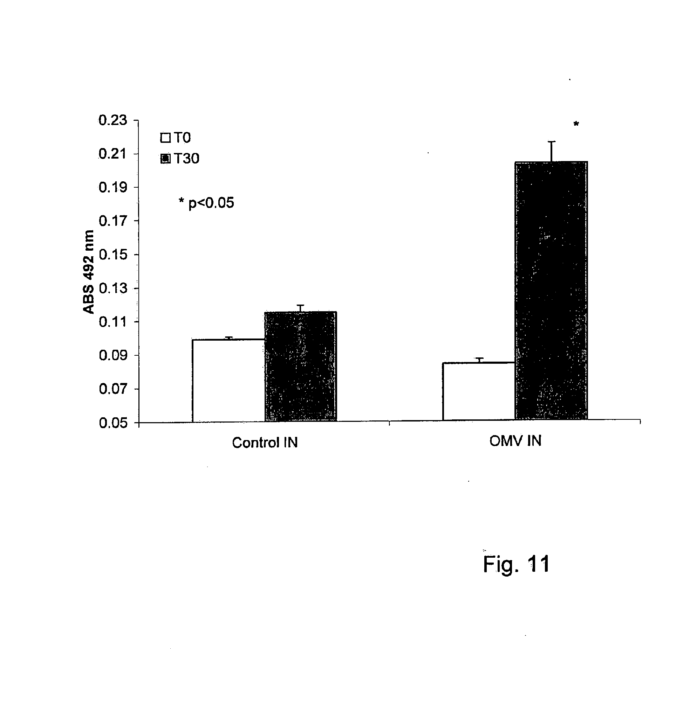

Balb/c mice were intra-nasally (IN), intra-gastrically (IG) or intra-muscularly (IM) immunized. In the case of the IN method, a concentration of 100 µg of proteins per mice of the cochlear structures was administered, while a concentration of 12 µg was used in the rest of the cases. The doses were separated by a period of 21 days in all cases. The vaccine VA-MENGOC-BC^{®} was employed as a positive control, being intra-muscularly administered at a concentration of 12 µg. Blood samples were taken from the animals 21 days after the second dose was administered and the titers of IgG1 and IgG2a present in the serum were determined through an ELISA test. In all of the cases considered (with the exception of the negative control cases), significant titers for IgG2a were obtained (p<0.05). These were at their highest value when the cochlear structures were administered intra-nasally. This indicates the inducement of a pattern of IgG, cellular Th1 type antibodies, especially favored by nasal inoculation (Fig. 11).

### Example 9. Subclasses of IgG in serum induced by immunization with outer membrane vesicles (OMV)

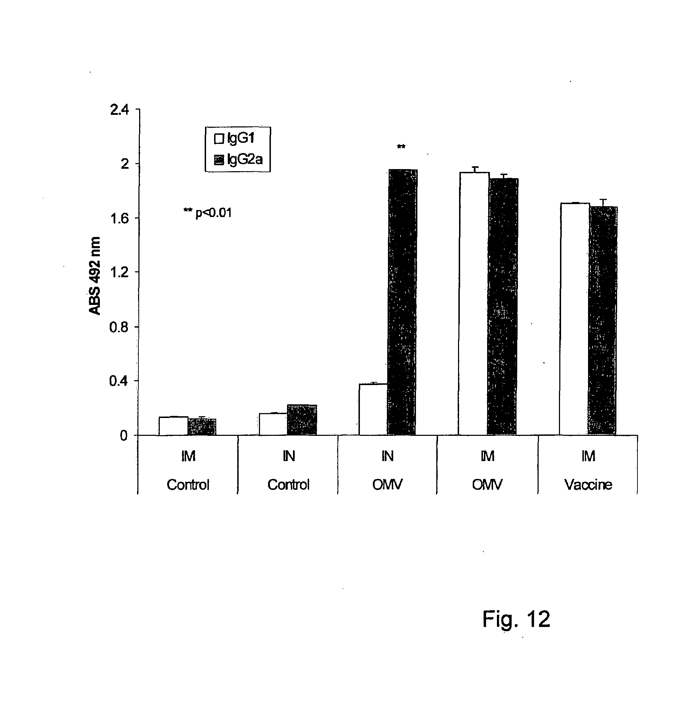

Balb/c mice were intra-nasally (IN) and intra-muscularly (IM) immunized with 12 µg of proteins per mice of outer membrane vesicles, in two doses separated by 21 day.The vaccine VA-MENGOC-BC^{®} was administered at the same concentration as a positive control. Blood samples were taken 21 days after the second dose was administered and the titers of IgG1 and IgG2a anti OMV present in the serum were analyzed through an ELISA test. In all of the cases considered, significant titers of IgG2a were induced by the outer membrane vesicles, indicating the inducement of a pattern of cellular Th1 type IgG antibodies. This was not the case with the negative IM or IN controls. A complete reveral of the pattern was observed in the case of IN inoculation, where the response was almost exlusively that of IgG2a (Fig. 12).

### Example 10. Thermal and acidic resistance of the cochlear strucutres obtained from outer membrane vesicles

The thermal resistance of the cochlear structures was evaluated by exposing the samples to a temperature of 60 °C for a period of 7 days. The resistance to acid was evaluated by exposing the samples to medium with a pH value of 1 for a period of 45 minutes. Following this, the treated and the control samples were used to intra-muscularly inoculate Balb/c mice with two doses of a concentration of 12 µg per mice, separated by 14 days. Blood samples were taken from the mice after 28 days following the start of the experiment and the serums were kept individually at -20°C until the time of their use. As can be observed, there were no significant differences (p<0.5) between the responses of anti-vesicle IgG induced in each of the animals and groups.

### Example 11. Production of IL12 in the U937 cell line stimulated exclusively with cochlear structures.

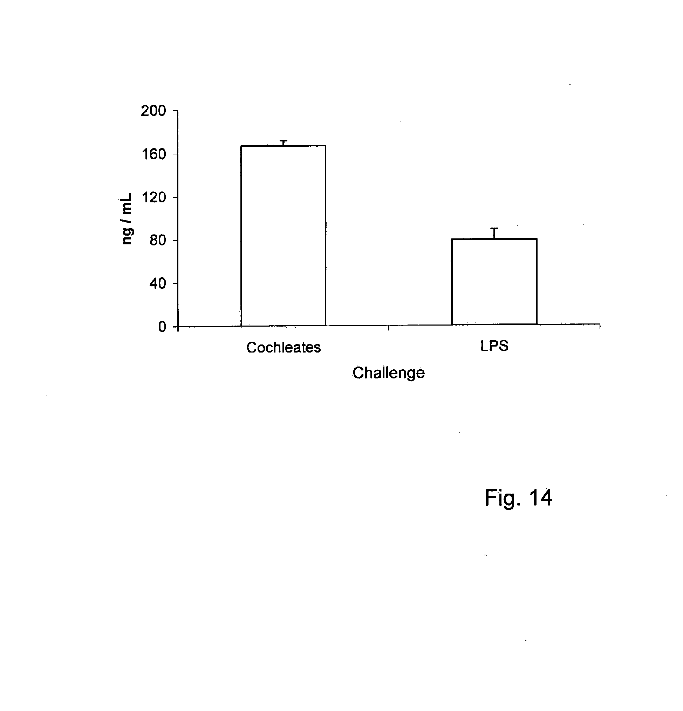

U937 cells were cultivated in RPMI 1640 supplemented with gentamicin at a concentration of µg/mL, L-glutamine (at 2 mM), sodium pyruvate (at 1mM), HEPES (at 15 mM) and fetal bovine serum (Sigma) at 10 %. These were differentiated into macrophages through a PMA treatment and were placed in flat-bottomed, 24-hole culture dishes, 5 x 10⁵ cells per hole. After 24 hours, the cochlear structures were added to them at a concentration of 250 ng/mL in the culture medium. After 24 hours of stimulus, the surviving cells were gathered and the presence of IL12 was determined through an sandwich-type ELISA test. The production of IL12 by the U937 cells stimulated by the cochlear structures was observed (Fig. 14).

### Example 12. Production of IL12 in the U937 cell line stimulated exclusively with outer membrane vesicles (OMV)

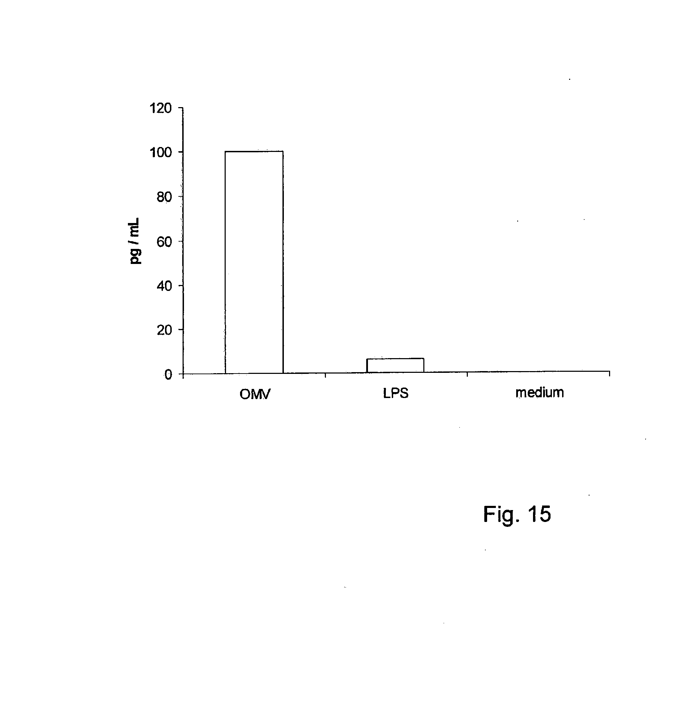

U937 cells were cultivated in RPMI 1640 supplemented with gentamicin at a concentration of µg/mL, L-glutamine (at 2 mM), sodium pyruvate (at 1mM), HEPES (at 15 mM) and fetal bovine serum (Sigma) at 10 %. These were differentiated into macrophages through a PMA treatment and were placed in flat-bottomed, 24-hole culture dishes, 5 x 10⁵ cells per hole. After 24 hours, the outer membrane vesicles were added to them at a concentration of 250 ng/mL in the culture medium. After 24 hours of stimulus, the surviving cells were gathered and the presence of IL12 was determined through an sandwich-type ELISA test. The production of IL12 by the U937 cells stimulated by the outer membrane vesicles was observed (Fig. 15).

### Example 13. Production of nitric oxide by the J774 murine macrophagyc cell line stimulated exclusively with cochlear structures

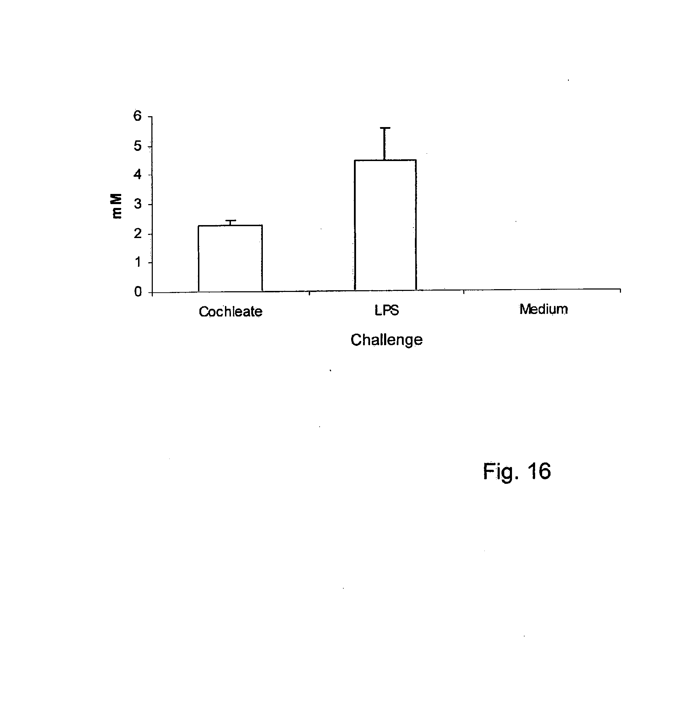

J774 cells were cultivated in a DMEN medium supplemented with gentamicin at a concentration of 50 µg/mL, L-glutamine (at 2 mM), sodium pyruvate (at 1 mM), HEPES (at 15 mM) and fetal bovine serum (Sigma) at 10 %, previously inactivated at 56 °C for 30 minutes. They were placed in flat-bottomed, 96-hole culture dishes, at a concentration of 1 x 10⁵ cells per hole and they were incubated for a period of 24 hours at 37°C and 5 % CO2. Following this, the adhering cells were incubated with 200 L of DMEN along with the cochlear structures at a concentrastion of 250 ng/mL. Other variants incubated with L-NMMA (at 1 µM), an inhibitor of the production of nitric oxide, were also included. The surviving cells were collected after 24 and 48 hours and analyzed for nitric contents using Greiss' reaction (Rockett, KA et a/., Infect. Immun. 1992, 60:3725-3730). A significant production of nitric oxide by the cells incubated with the cochlear structures was observed. This production was inhibited by the use of L-NMMA (Fig. 16).

### Example 14. Production of nitric oxide by the J774 murine macrophagyc cell line stimulated exclusively with outer membrane vesicles (OMV)

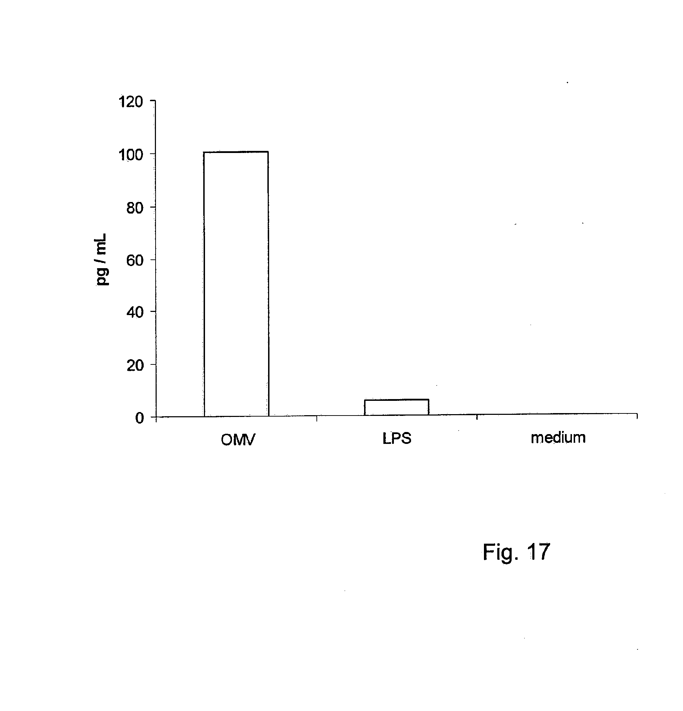

J774 cells were cultivated in a DMEN medium supplemented with gentamicin at a concentration of 50 µg/mL, L-glutamine (at 2 mM), sodium pyruvate (at 1mM), HEPES (at 15 mM) and fetal bovine serum (Sigma) at 10 %, previously inactivated at 56 °C for 30 minutes. They were placed in flat-bottomed, 96-hole culture dishes, at a concentration of 1 x 10⁵ cells per hole and they were incubated for a period of 24 hours at 37°C and 5 % CO2. Following this, the adhering cells were incubated with 200 L of DMEN along with the outer membrane vesicles at a concentration of 250 ng/mL. Other variants incubated with L-NMMA (at 1 µM), an inhibitor of the production of nitric oxide, were also included. The surviving cells were collected after 24 and 48 hours and analyzed for nitric contents using Greiss' reaction (Rockett, KA et al., Infect. Immun. 1992, 60:3725-3730). A significant production of nitric oxide by the cells incubated with the outer membrane vesicles was observed, greater than that induced by the LPS utilized as a control. This production was inhibited by the use of L-NMMA (Fig. 17).

### Example 15. Stimulation of human dendryte cells by the cochlear structures

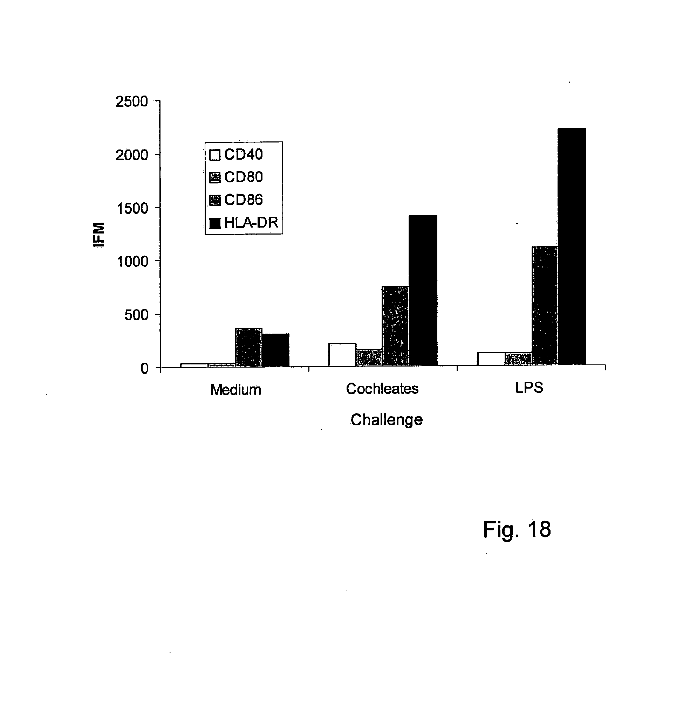

Blood was extracted and the peripheral mononuclear cells were purified using ficoll. The cells were cultivated at 10 x 10¹⁸ cells per mL in the presence of LPS or cochlear structures and the activation of dendrite cells was determined through Flow Cytometry. As can be observed in Fig. 18, the dendrite cells were activated (determined through the expression of co-stimulant molecules, such as CD40, CD80 and CD86), and the expression of MHC molecules was increased. This demonstrates the adjuvant nature of these structures.

### Example 16. Reduction of indurations in Balb/c immunized with cochlear structures containing amastygotes of Leishmania major and challenged with the same protozoan organism

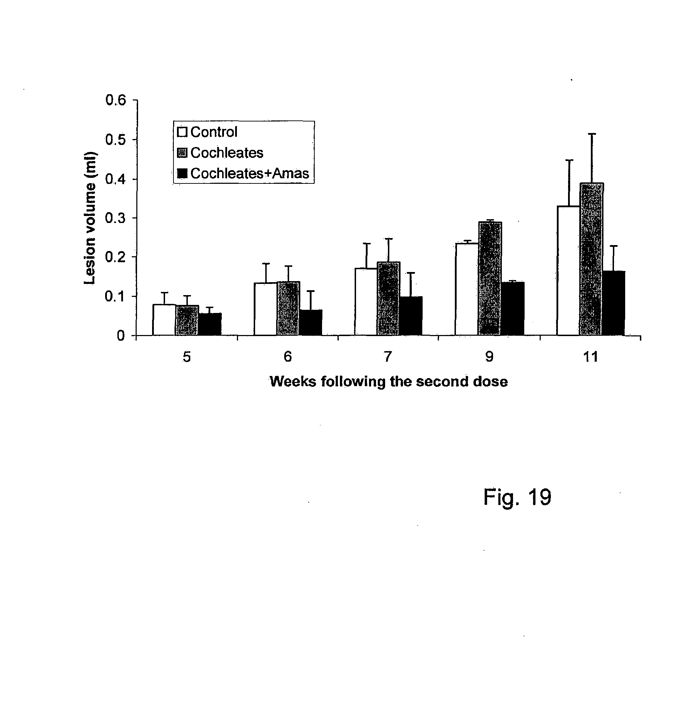

The inclusion of amastygotes derived from L. *major* was achieved by including the semi-purified antigens in the first steps of the formation of the cochlear structures. The amount of detergent used was adjusted to the total protein content and the total concentration of proteins was maintained at a range of 5-6 mg/mL. The ratio of vesicular proteins to the new antigens included was that of 12:1. The formation of cochlear structures was verified through optical and electronic microscopy. The inclusion of proteins from L. *major* was also verified through electrophoresis in polyacrilamyde gels tinted with Coomassie Blue. Balb/c mice were intra-muscularly immunized with 12 µg of the cochlear structures in 2 doses separated by 21 days. The cochlear structures were inoculated at the left posterior extremity. After 21 days following the second dose, the mice were infected with 3 x 10⁶ promastygotes at the same extremity inoculated. The promastygotes were obtained from the stationary phase of the cultures grown in a DMEN medium over a solid agar-blood medium. The volume of lesions was stimulated weekly starting at the fourth week following the infection. A significant reduction in the size of the lesions was observed in the group immunized with the cochlear structures containing antigens of *L*. *major.* This demonstrates the adjuvant character of this structure (Fig. 19).

### Example 17. Inclusion of plasmidic DNA with a fluorescent green protein.

Purified plasmids containing the gene of the fluorescent green protein under a CMV promoter were included in the initial solution used for obtaining the cochlear structures, following the same steps for obtaining said structures described in Example 1. The ratio of plasmids to vesicular proteins was adjusted to 1:100. The inclusion of the plasmids was checked using electrophoresis in agar gel at a 1 % concentration of the cochlear structures previously incubated at 37°C for 30 minutes, after adding EDTA to a quantity of 2 mM in order to provoke the freeing of plasmids inside them. The gels were tinted with ethidium and were observed under ultraviolet light. The presence of plasmids was detected only in the cochlear structures that contained them after being treated with EDTA. Following this, a transfection trial was carried out in the J774 cell line using these structures. After 2 hours of incubation, the cochlear structures were eliminated from the culture medium. The inspection of the cells under fluorescence 24 hours later revealed the presence of numerous cells with fluorescent signals in the cytoplasm.

### Example 18. Strenghening of cellular response through the conjugation of outer membrane vesicles

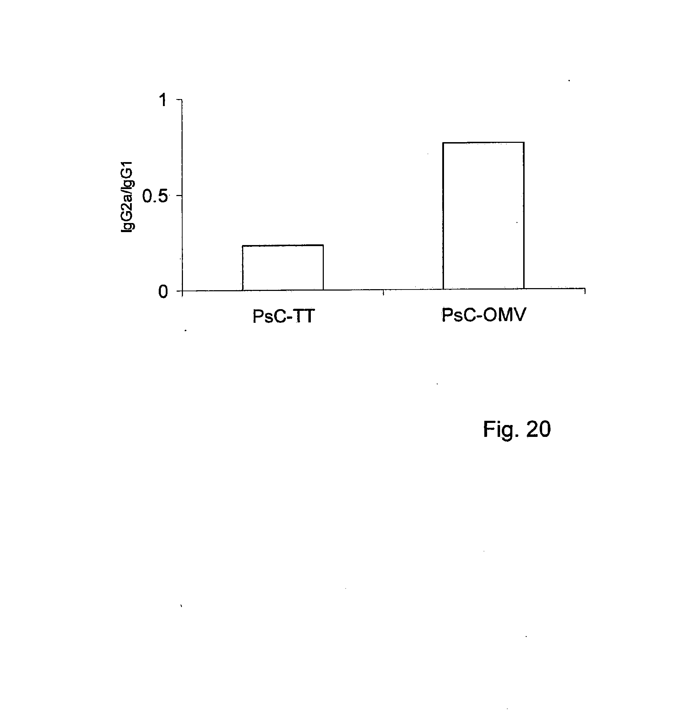

The polysaccharide (PsC) of serum group C of *Neisseria meningitidis* was conjugated with the tetanus toxoid (TT) or to outer membrane vesicles (OMV) of *N. meningitidis* serum group B. Balb/c mice were given three, intra-peritoneous inoculations (at days 0, 14 and 28) containing 10 µg of combined PsC. Blood samples were taken from the animals before and 42 days following the immunization. The responses of IgG and its subclasses in the serum were determined. The strongest response of IgG2a found in the group conjugated to outer membrane vesicles is indicative of a better cellular response obtained in comparison to that induced in the group conjugated with the tetanus toxoid (TT) (Fig. 20).

### Example 19. Strengthening of the response of anti-polysaccharide Vi antibodies through conjugation

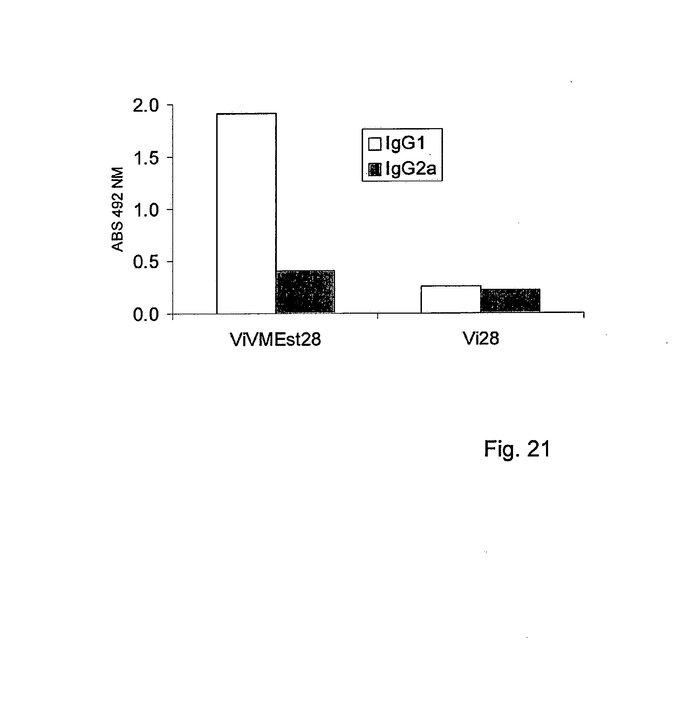

The polysaccharide Vi from *Salmonella tiphy* was conjugated to outer membrane vesicles (OMV) of S. *tiphy.* Balb/c mice were intra-peritoneously immunized with two doses (administered at day 0 and 28) containing 10 µg of the Vi. Blood samples were taken from the animals before and 42 days following the inoculation. The responses of IgG and its subclasses in the serum were determined. Conjugation increases and positivizes the response against the Vi polysaccharide and a response offered by IgG2a is detected (Fig. 21).

### Example 20. The possibility of including different concentrations of molecular structures associated to pathogens in cochlear structures

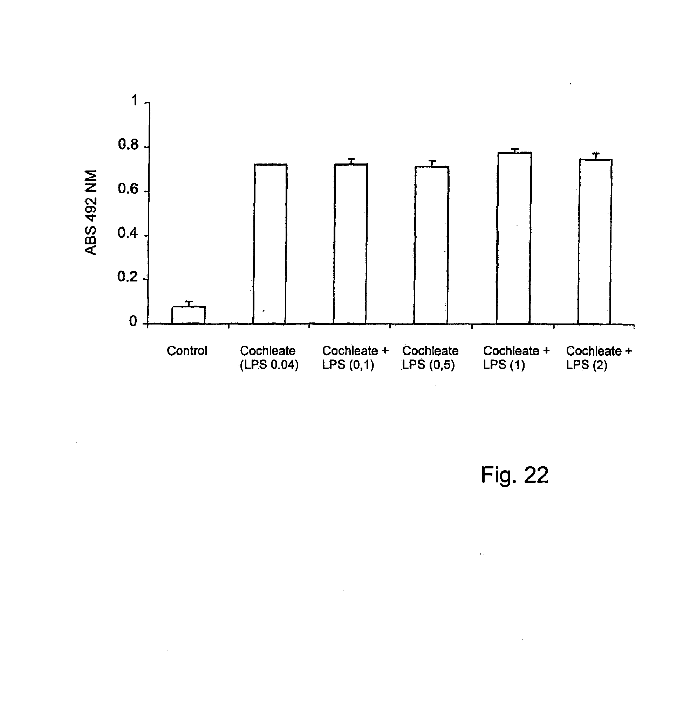

Different quantities of LPS derived from *Neisseria meningitidis* B were experimented with for the inclusion of different concentrations of molecular strucutres associated to pathogens in the cochlear structures. The ratios of LPS concentration to protein concentration used for the immunization of the mice were: 0.05:12, 0.5:12, 1:12, and 2:12. The formation of cochlear structures was verified through optical microscopy, which determined a ratio of 1:12 as the maximum ratio in which the LPS may be introduced without affecting the formation of the cochlear structures. Larger quantities visibly affect the formation of the structures, resulting in the formation of aggregates. All of the obtained variants were administered to Balb/c mice in two doses, separated by 21 days, of 12 µg of proteins per mice. The titers of anti-vesicle IgG were determined. No difference between the titers resulting from the use of the different variants were observed. The experiment guarantees the possibility of incorporating different LPS in these structures, however. (Fig. 22).

### Example 21. Effectiveness of the proposed method in the final steps of the production of cochlear structures

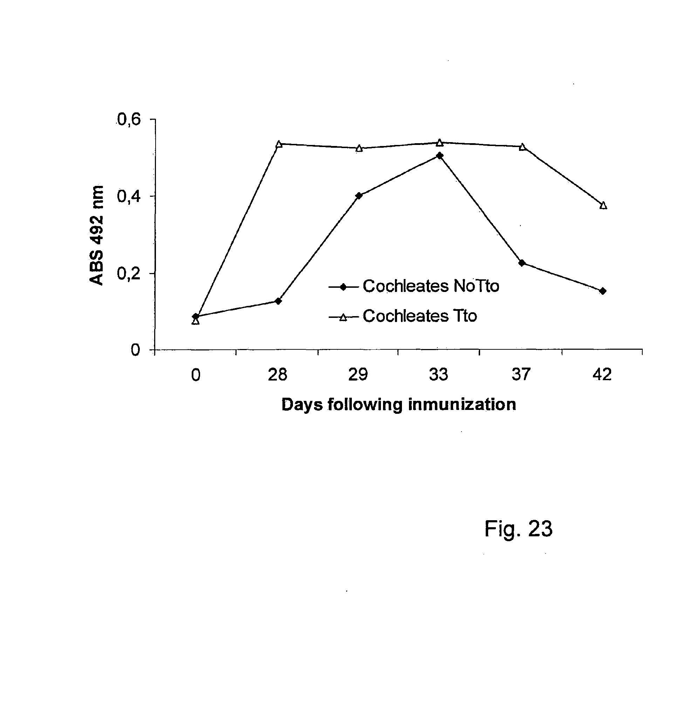

Cochlear structures treated with light sonication (Tto) in a water bath for 45 minutes at 20°C, as well as untreated structures, were employed. Balb/c mice were intra-nasally immunized with 100 µg of proteins per mice in two doses separated by 21 days. Saliva samples were taken 9 days after the last dose was administered and the responses of IgG against vesicles of outer membrane (OMV) were evaluated through an ELISA test. The immunological responses took place significantly earlier or operated for a longer period of time in those animals treated with the structures, as shown in Figure 23.

### Example 22. Response of IgA against outer membrane vesicles in serum, saliva and vagina, induced by perenteral and mucosal immunization and evaluated through an ELISA test

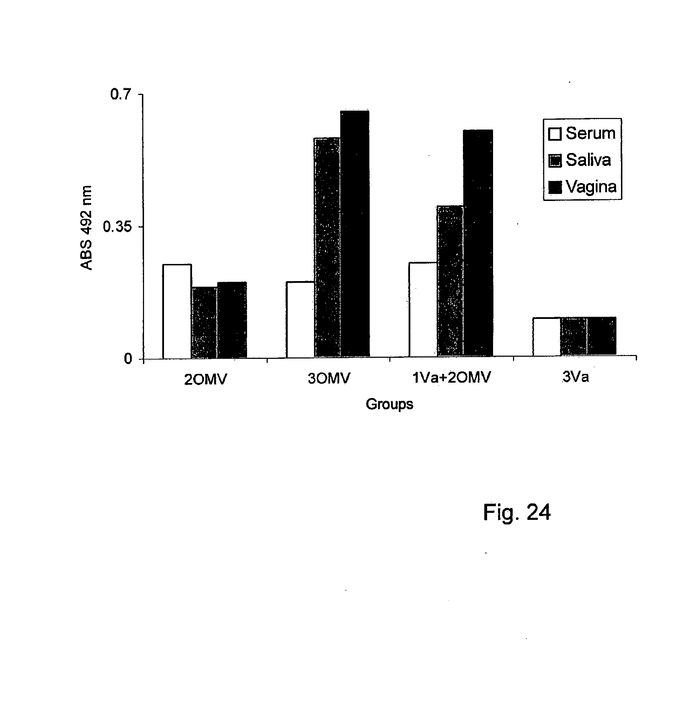

Balb/c mice were intra-nasally (IN) immunized with 2 or 3 doses of outer membrane vesicles (OMV), intra-muscularly administered 3 doses of the vaccine VA-MENGOC-BC® as a control, and a combination of 1 and 2 doses of the vaccines administered using the IM and IN methods, respectively. Each mouse was administered 12 µg of protein at times 0, 21 and 42. The serums were taken after 15 days and the saliva and vaginal fluid after 9 days following the last dose. The results were evaluated through an ELISA test. As can be observed, nasal immunization induces a small increase of the IgA at the level of the serum, while the immunization with the vaccine using the IM method does not. The mucosal response depended on the number of doses: two doses did not induce a response, while 3 doses resulted in a response of anti-vesicle IgA. Finally, 2 doses administered nasally proved effective in animals that received a stimulus (one dose) of the vaccine administered intra-muscularly (Fig. 24).

### Advantages of the proposed solution:

1. The outer membrane vesicles are extracted from live organisms, allowing for the selection of constituents during the extraction process of the outer membranes, which constitute the first defense barrier in the contact between host and pathogen, making these constituents suited for the protection of both animals and humans;
2. during the extraction process, other proteins of interest, be them natural or recombining, may be included;
3. vesicles of outer membrane extracted from live organisms are more stable than artificially constructed liposomes, and can remain intact for a number of months, even years, without suffering significant alterations that can affect the formation of future cochlear structures;
4. the Th1 cellular response induced in animals and humans makes these adjuvants effective, not only in adults and children, but also during breast-feeding;
5. the cochlear structures formed are thermo-resistant, something which can prove useful in solving the problems associated with the cold chain of a number of vaccines, be it through their formulation as an adjuvant or their development from outer membrane vesicles and cochlear structures;
6. the cochlear structures formed are resistant to both bases and acids, something to be kept in mind when considering orally administered vaccines;
7. the antigens are incorporated in the structures during the production process, making the final product thermo, acid and base resistant;
8. the versatility of the antigens that may be included, be them soluble or particulated, including nucleic acids, allows for the production of a great many vaccines, including multiple ones;
9. the cochlear structures contain molecular structures associated to pathogens, and others may be incorporated at will in order to increase its adjuvant and immunological effectiveness, allowing us to reduce the potential toxicity of some of these structures and thus their inflammatory effects;
10. the cochlear structures induce earlier, stronger and longer-lived reactions *in vivo*;
11. the cochlear structures induce, *in vitro,* better responses at the level of cytokinins which induce patterns of cellular immune responses;
12. the structures preserve the properties of artificial cochleates (the efficient incorporation of hydrophobic antigens, slow deployment system, the content of calcium as an essential mineral, the reduction of lipidic oxidation, freeze drying, etc.), but it is superior to these in immonogenicity, its inclusion of molecular structures associated to pathogens and in its capacity to induce a Th1 pattern, including a T cytotoxic response and the use of lipids and cholesterol, derived from animal serum, is avoided.

### Brief description of figures

Figure 1. Method for producing cochleates described in Gould-Fogerite et al. U.S. Pat. No. 5,643,574, July 1, 1997.
Figure 2. Simplified method for obtaining cochlear structures, object of the present invention.
Figure 3. Electron microscopy of a cochlear structure.
Figure 4. A: Electrophoresis in acrylamyde gel at 12.5 % tinted with Coomassie Blue of the proteins present in the vesicles of outer membrane. B: Western Blot of the proteins present in vesicles of outer membrane and the cochlear structures, using a human serum of a high titer of antibodies reactive against vesicles of outer membrane.
Figure 5. Serum responses of IgG against outer membrane vesicles in mice parenterally immunized with VA-MENGOC-BC^{®} or cochlear structures, evaluated by an ELISA test.
Figure 6. Serum responses of IgG against outer membrane vesicles in mice intra-muscularly immunized with VA-MENGOC-BC^{®} or outer membrane vesicles, evaluated by an ELISA test.
Figure 7. Serum responses of IgG against outer membrane vesicles in mice intra-gastrically (IG) or intra-nasally (IN) immunized with outer membrane vesicles, evaluated by an ELISA test.
Figure 8. Serum responses of IgG against outer membrane vesicles in mice intra- intra-nasally (IN) immunized with outer membrane vesicles, evaluated by an ELISA test.
Figure 9. Response of IgA in saliva against outer membrane vesicles in mice intra-gastrically or intra-nasally immunized with cochlear structures, evaluated by an ELISA test.
Figure 10. Response of IgA in saliva against outer membrane vesicles in mice intra-nasally immunized with outer membrane vesicles, evaluated by an ELISA test.
Figure 11. Results of the subclasses of IgG reactive against outer membrane vesicles in animals immunized with cochlear structures, evaluated by an ELISA test.
Figure 12. Results of the subclasses of IgG reactive against outer membrane vesicles in animals immunized with outer membrane vesicles, evaluated by an ELISA test.

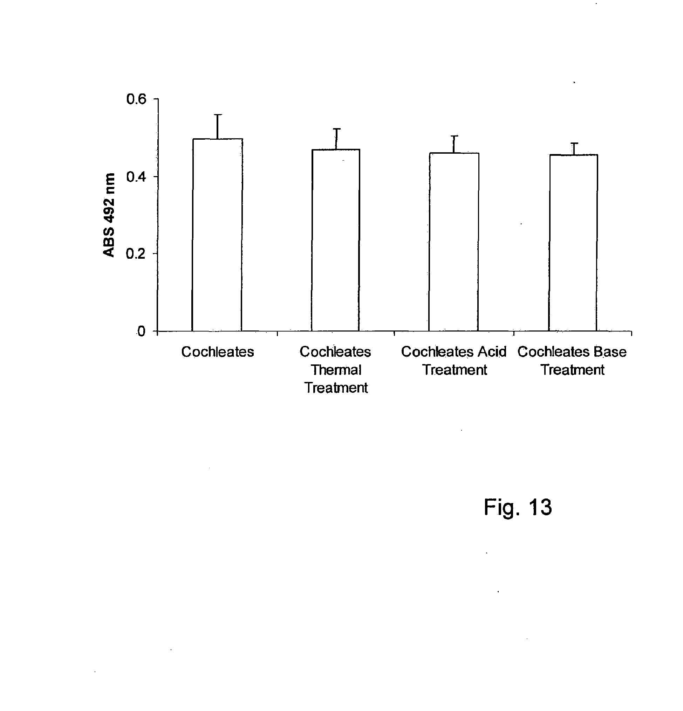

Figure 13. Results of thermo and acid resistance of the cochlear structures, evaluated by an ELISA test.
Figure 14. Evaluation of the production of IL12 by U937 cells stimulated with the cochlear structures.
Figure 15. Evaluation of the production of IL12 by U937 cells stimulated with outer membrane vesicles derived from *Neisseria meningitidis.*
Figure 16. Production of nitric oxide by J774 cells incubated with cochlear structures.
Figure 17. Production of nitric oxide by J774 cells incubated with outer membrane vesicles derived from *Neisseria meningitidis.*
Figure 18. Stimulation of human dendrite cells with cochlear structures.
Figure 19. Results of the indurations in animals immunized with cochlear structures containing amastygotes and challenged with *Leishmanais major.*
Figure 20. Results of the adjuvant effect of the conjugation of polysaccharide with outer membrane vesicles of *Neisseria meningitidis.*
Figure 21. Results of the adjuvant effect of the conjugation of polysaccharide with outer membrane vesicles of *Salmonella tiphy.*
Figure 22. Results of the incorporation of molecular structures associated to pathogens.
Figure 23. Results of the effect of sonication on the response induced by cochlear structures.
Figure 24. Results of the strengthening of the mucosal response after IN inoculation following an initial intra-muscular stimulus.

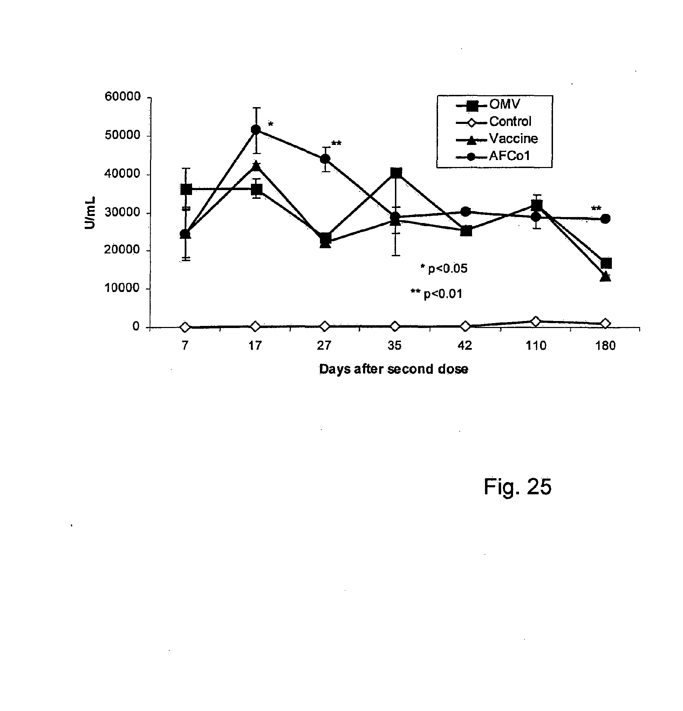

Figure 25. Kinetic of IgG anti-OMV response potenciates by the cochleate structure (AFCo1). The IgG response anti-OMV was determined in the sera of mice immunized intramuscularly with OMV, AFCo1 or VA-MENGOC-BC^{™} vaccine. AFCo1 induced an IgG response significantly higher and durable.

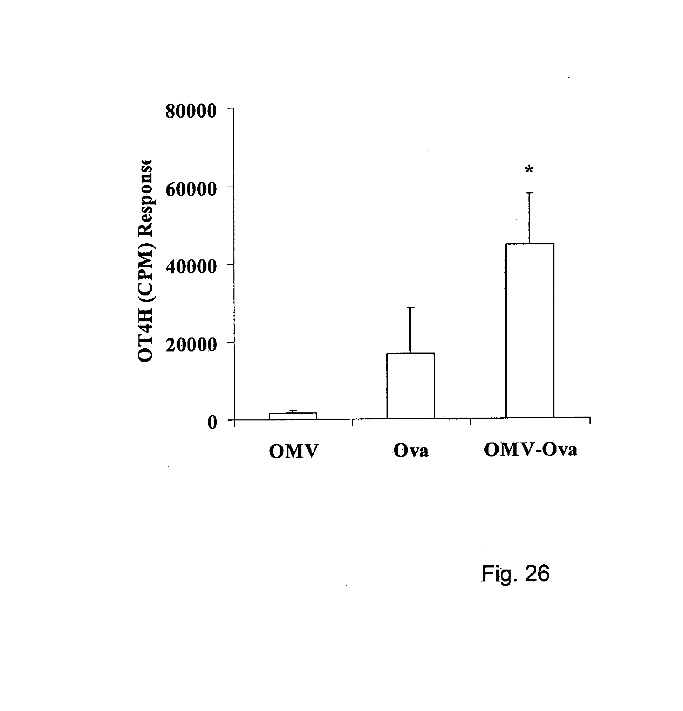

Figure 26. DC can process OVA peptides from OVA included in the outer membrane vesicles (OMV) (OMV-Ova) for MHC-II presentations. IL-2 production from the OT4H T-hybridoma cells after co-incubation with OMV-Ova was quantified by [³H]thymidine incorporation by the IL-2-dependent CTLL cell line. Data are presented as the mean + SD of three different experiments.
   *significantly different from other groups.

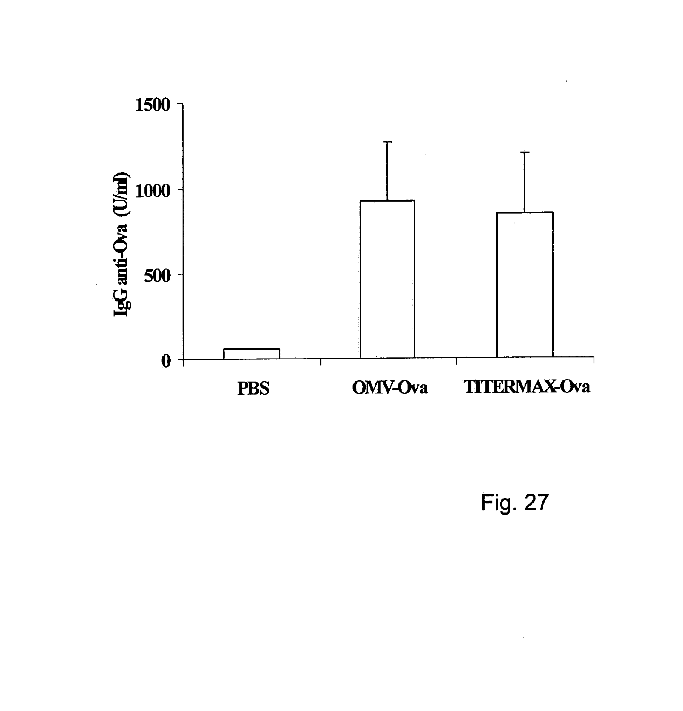

Figure 27. IgG anti-Ova response in mice immunized with OMV-Ova. C3H/HeN mice were immunised with two doses of OMV-Ova 5 mg/ml, phosphate buffer solution or Titermax-Ova emulsion (positive control). Sera were collected 21 days after the first dose and evaluated by ELISA. Data show the average IgG concentration of five mice per group ± the standard deviation and are representative of three different assays.

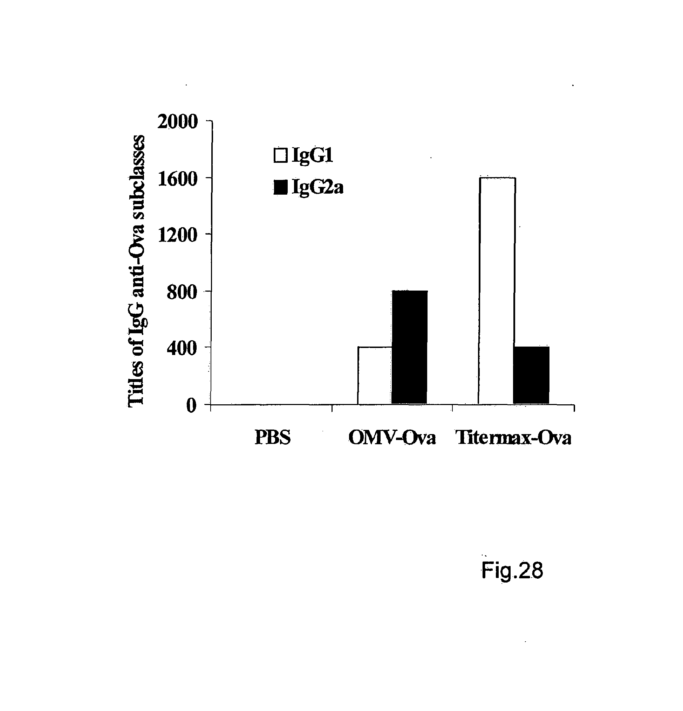

Figure 28. IgG anti-Ova subclasses response in mice immunised with OMV-Ova. C3H/HeN mice were immunised with two doses of OMV-Ova 5 mg/ml, phosphate buffer solution or Titermax-Ova emulsion (positive control). Sera were collected 21 days after the first dose and evaluated by ELISA. Data show the IgG1 and IgG2a titles in pool sera from five mice per group and are representative of three different assays.

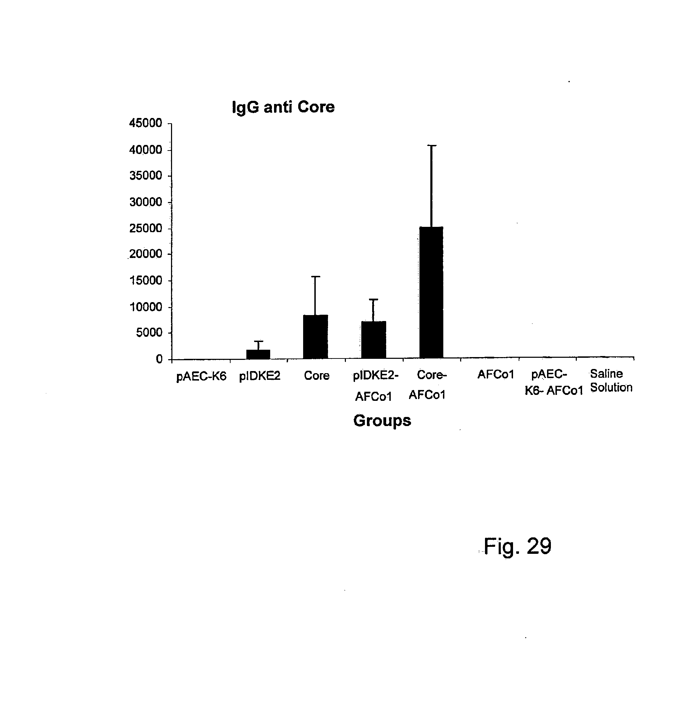

Figure 29. IgG response against Core protein of the Hepatitis C Virus (VHC) induced by intramuscular immunization of animals, evaluated by ELISA. The IgG anti Core protein was determined in the sera of immunized animal by intramuscular route with three doses (0, 3, and 7 weeks) and the samples were taken two weeks after the last dose. As it is showed the incorporation of the two antigens (Core and Capside proteins) in AFCo1 induced responses significantly higher.

## Claims

1. Vaccine composition containing vesicles obtained from the outer membrane of live organisms and, optionally, one or more antigens, as well as an adequate excipients, preferably wherein said outer membrane vesicles are composed of proteins, lipids and molecular structures associated to pathogens.

2. Vaccine composition according to Claim 1, with said molecular structures associated to pathogens found at a concentration between 1 % and 7 % of the protein weight of the structure.

3. Vaccine composition according to Claim 1, with said molecular structures associated to pathogens selected from the group consisting in lipopolysaccharide, peptydoglycane, teicoic acid, flagellin and lipophosphoglycane.

4. Vaccine composition according to Claim 1, **characterized by** the fact that the live organism supplying the vesicles of outer membrane used to form the cochlear strucutres is a bacterium, a protozoan or an animal cell, preferably said vaccine composition is **characterized by** the fact that said bacterium is a Gram negative or a Gram positive.

5. Vaccine composition according to Claim 4, **characterized by** the fact that said Gram negative bacterium is of *Neisseria, Haemophilus, Salmonella, Vibrio, Pseudomona* or *Shigella* genus.

6. Vaccine composition according to Claim 4, **characterized by** the fact that said Gram positive bacterium is of the *Streptococcus* or *Staphylococcus* genus.

7. Vaccine composition according to Claim 4, **characterized by** the fact that said live organism is a protozoan organism from the *Leishmania* genus.

8. Vaccine composition according to Claim 4, with the outer membrane vesicles derived from a tumor cell.

9. Vaccine adjuvant containing vesicles extracted from the outer membrane of live organisms, preferably wherein said outer membrane vesicles are composed of proteins, lipids, and molecular structures associated to pathogens.

10. Vaccine adjuvant according to Claim 9, with said molecular structures associated to pathogens found at a concentration between 1 % and 7 % of the protein weight of the structure.

11. Vaccine adjuvant according to Claim 10, with said molecular structures associated to pathogens selected from the group consisting in lipopolysaccharide, peptydoglycane, teicoic acid, flagellin and lipophosphoglycane.

12. Vaccine adjuvant according to Claim 9, **characterized by** the fact that the live organism supplying the vesicles of outer membrane used to form the cochlear structures is a bacterium, a protozoan or an animal cell, preferably, wherein said vaccine adjuvant is **characterized by** the fact that said bacterium is a Gram negative or a Gram positive.

13. Vaccine adjuvant according to Claim 12, **characterized by** the fact that said Gram negative bacterium is of *Neisseria, Haemophilus, Salmonella, Vibrio, Pseudomona* or *Shigella* genus.

14. Vaccine adjuvant according to Claim 12, **characterized by** the fact that said Gram positive bacterium is of the Streptococcus or Staphylococcus genus.

15. Vaccine adjuvant according to Claim 11, **characterized by** the fact that said live organism is a protozoan from the Leishmania genus.

16. Vaccine adjuvant according to Claim 11, with the outer membrane vesicles derived from a tumor cell.

17. A method for obtaining cochlear structures from vesicles found in the outer membrane of live organisms, composed of the following steps:
a. preparation from outer membrane vesicles, of a solution with a total protein concentration between 3 and 6 mg/mL, to which a non-ionic detergent is added at a concentration 10 times that of the proteins.
b. should one wish to incorporate other antigens of interest or molecular structures associated to pathogens, these are added to the solution prepared in a), homogenizing it at 0.2 to 2.7 µg for each 3 to 9 µg of protein for the antigens and from 1 to 30 % of the protein concentration for the molecular structures.
c. following this, the solution of steps a) and b) is filtered through a membrane with a pore size of 0.2 µm, with the aim of sterilizing and eliminating vesicle aggregates yet found in it.
d. a rotational dialysis or a tangential filtration is then executed, against a solution containing concentrations of a multivalent ion, particularly Ca²⁺, Zn²⁺, or Mg²⁺, between 2.5 and 6.5 mM, at conditions buffered at pH 7.4 ± 0.2.
e. finally, the cochlear structures obtained are mechanically treated, submitted, especially, to sonication in a water bath at a temperature between 15 °C and 25 °C for a period of 45 minutes, in order to homogenize the size of the particles.

18. Vaccine composition according to any one of claims 1 to 8, administered mucosally, parenterally, or through a combination of both methods.

19. The adjuvant according to Claims 9 to 16, administered mucosally, parenterally, or through a combination of both methods.
